# EUROPEAN PATENT SPECIFICATION

(11) **EP 0 639 086 B1**
(45) Date of publication and mention of the grant of the patent: **08.08.2001**
(21) Application number: 93910849.4
(22) Date of filing: 28.04.1993
(51) Int. Cl.: A61L 15/14

(54) **ORTHOPEDIC CASTS**
ORTHOPÄDISCHE GIPSVERBÄNDE
PLATRES ORTHOPEDIQUES

(30) Priority: 29.04.1992 US 875776
(43) Date of publication of application: 22.02.1995
(73) Proprietor: LANDEC CORPORATION, Menlo Park, CA 94025 (US)
(72) Inventor: STEWART, Ray, F., Redwood City, CA 94062 (US); YOON, Valentine, Y., Redwood City, CA 94065 (US); LARSON, Andrew, W., Palo Alto, CA 94306 (US); ROSS, Thomas, W., Los Gatos, CA 95030 (US); GREENE, Lawrence, C., Boulder Creek, CA 96006 (US); KAMP, David, A., Sunnyvale, CA 94087 (US); SCHMITT, Edward, E., Palo Alto, CA 94306 (US)
(74) Representative: Hall, Robert Leonard
(86) International application number: US9303962
(87) International publication number: WO9321967

(56) References cited:
- EP-A- 0 169 037
- EP-A- 0 317 180
- WO-A-90/13420
- WO-A-91/09909
- WO-A-93/07194
- FR-A- 2 417 524
- GB-A- 2 140 429
- US-A- 4 951 656

## Description

This invention relates to orthopedic casts.

It is well known to make orthopedic casts, splints, supports, braces, shields, wound covers, and like orthopedic devices (all of which are referred to herein simply as "casts") from calcined gypsum (Plaster of Paris) or from a polymer which is formed into shape while hot and hardens on cooling. The term "casting composition" is used herein to denote a polymeric composition which can be formed into shape while hot and hardens on cooling. The casting composition can comprise a single polymer or a mixture of two or more polymers, and can contain additional ingredients such as inorganic fillers. The known casts can contain a flexible support, which may be elastic, for example a fabric or a foam sheet. Reference should be made for example to US Patent Nos. 3,692,023 (Phillips), 3,728,206 (Buese), 3,809,600 (Larson), 4,231,356 (Usukura), 4,404,333 (Watanabe), 4,473,672 (Green), 4,668,563 (Buese), 4,784,123 (Robeson), 4,912,174 (Grouiller), and 4,946,726 (Sandvig), British Patent No, 1,522,399 (Hexcel) and European patent publication 0,110,860 (Luxilon). However, the known casts do not provide an entirely satisfactory combination of moldability at temperatures which are well tolerated by the recipient of the cast, rapid hardening, and, after hardening, adequate strength, light weight, good breathability, water resistance, and transparency to x-rays.

We have discovered a number of important improvements in casting compositions and in the construction, application and use of orthopedic casts, especially on human patients, but also on animals. These discoveries can also be utilized in equivalent and related fields, where the same or similar casts are applied to inanimate substrates and where the casting compositions are used for molding generally.

In a first aspect, the present invention provides a heat-recoverable cast which comprises
(1) a recoverable fabric support which is in a stretched condition; and
(2) a rigid casting composition which
   (a) maintains the support in its stretched condition, and
   (b) comprises a casting polymer having a crystalline melting point Tₘ;
whereby the cast
(A) at temperatures below Tₘ, has a first stable configuration,
(B) when heated to a temperature above Tₘ in the absence of restraint, becomes moldable and recovers so that at least one dimension thereof decreases from a first value x to a second value y which is at most 0.95 x, and
(C) when cooled to a temperature below Tₘ after being molded, adopts a second, stable configuration.

In a second aspect, the invention provides a process for making an orthopedic cast as defined in the first aspect of the invention by applying the casting composition, while it is molten, to the fabric support.

### Definitions. Abbreviations and Measurements

In this specification, parts, amounts and percentages are by weight. Temperatures are in °C. Molecular weights are weight average molecular weights expressed in Daltons and are determined by gel permeation chromatography (GPC) in tetrahydrofuran (THF). First order transition points (often referred to as melting points), glass transition points, and heats of fusion are determined by a Differential Scanning Calorimeter (DSC) using the second heat cycle and a heating rate of 10°C/minute. The peak of the DSC curve is referred to as Tₘ. Crystallization temperatures are determined by a DSC at a cooling rate of 10°C/minute.

The "power" of a material is defined as the force required to stretch a 1-inch (2.54 cm) wide sample of the material to twice its original length. Melt Index values are measured by ASTM D1238 (modified). Tensile modulus values are calculated according to ASTM D638 after testing the material by ASTM D1708 at a crosshead speed of 1 inch/minute (2.54 cm/minute). Strength values given herein are the tensile modulus of the material (psi) multiplied by the thickness of the material (inch). Strength values for casts above Tₘ are measured after heating the material to (Tₘ +10)°C and allowing it to equilibrate at that temperature for 5 minutes. Strength values for molded casts are measured at 25°C after heating the cast to at least (Tₘ +10)°C, allowing the cast to recover without any restraint (if it is heat-recoverable), cooling it to 25°C and allowing the cast to equilibrate at 25°C for 1 hour. Air flow permeability (AFP) values given herein are measured as follows. An air gun is secured in a jig, and an anemometer is placed in a reference position at the center of the air stream at a distance of 5 inch (12.7 cm) from the tip of the gun. The gun is adjusted so that the air is cold and the air flow recorded by the anemometer is about 1700 ft/min (570 m/min). A flat sample of the cast (or a corresponding sample -- see below) in the shape of a circle 2 inch (5.1 cm) in diameter, or a larger sample, is placed across the air flow at right angles to the air flow, at a distance of 5 inch (13 cm) from the tip of the gun. The air flow is recorded by the anemometer at three different spots within about 0.5 inch (1.2 cm) of the reference position, and is averaged. The AFP of the cast is the average air flow, expressed as a percentage of the air flow without the cast. Since the sample of the cast must be flat, and the casts, both before and after molding, are usually not flat, it is usually necessary to measure AFP values on corresponding samples, i.e. on sheets obtained by flattening out a portion of the cast, taking care not to change its porosity, or on sheets which are flat but which have otherwise been prepared in the same way as the cast. For example, after measuring the AFP of an unused sample using cold air, the air gun can be adjusted to provide hot air which will effect recovery of the sample, and the AFP of the recovered sample can be measured.

### Heat-recoverable Casts

The casts of the invention are, before use, heat-recoverable. Their heat-recoverability results at least in part from the presence in the cast of an elastomeric support which is maintained in a stretched condition by the rigid casting composition, and recovers towards an undeformed configuration when the casting composition is softened by heating. However, the casts can also be heat-recoverable in part because the casting polymer, and/or a support which forms part of the cast, is composed of a crosslinked polymer which has been deformed above its melting point, and cooled in the deformed condition; such a material, when heated above its melting point, will tend to return to its undeformed condition.

The available recovery of a cast will depend on the extent of the deformation put into it. The cast, if heated to a temperature above Tₘ, in the absence of any restraint, will recover so that at least one dimension thereof decreases from a first value *x* to a second value *y*, where *y* is at most 0.95*x*, particularly at most 0.75*x*. It is generally unnecessary for *y* to be less than 0.38*x*, and in most cases *y* is more than 0.45*x*.

### Casting Polymers

The casting polymers used in this invention preferably have a Tₘ of at least 40°C, particularly at least 45°C, to ensure that the cast does not soften under normal atmospheric conditions. It is also preferred that Tₘ is no more than 60°C, particularly no more than 55°C, so that the casting polymer will soften and can be molded at temperatures which do not cause distress to human patients. Melting preferably takes place over a range of less than 20°C, particularly less than 15°C, especially less than 10°C, more especially less than 5°C. If the casting polymer is a polyolefin, it preferably consists essentially of a single tactic form, i.e. is wholly atactic or syndiotactic or isotactic, so that its melting point is sharp. Particularly when using polymers with Tₘ's of 40-60°C, it is preferred that recrystallization should take place rapidly on cooling from above Tₘ to below Tₘ, so that the patient does not have to remain still for an extended time while the cast hardens; we have found that excellent results are obtained in practice if, when the cast is heated above Tₘ and then cooled to (Tₘ - 10)°C at a rate of 10°C/min, the casting polymer recrystallizes not more than 2 minutes, preferably not more than 1 minute, after it has cooled to Tₘ. The recrystallization temperature of the polymer is preferably about 35 to 55°C. Casting polymers having Tₘ's above 60°C, e.g. some polycaprolactone-based compositions, can be used, but greater care is needed when using them, since it is usually necessary (in order to prevent distress to the patient) to effect at least some of the molding of the cast at a temperature which is below Tₘ but at which the polymer has not yet recrystallized. For those polymers, therefore, it may be disadvantageous for recrystallization to take place very rapidly on cooling from above Tₘ to below Tₘ.

Preferred casting polymers for use in the present invention comprise crystalline polymers in which the crystallinity results exclusively or predominantly from side chains which are attached to the polymer backbone. Such polymers are often referred to as side chain crystallizable polymers, or SCC's, and include polymers containing units derived from (or derivable from) one or more monomers such as substituted and unsubstituted acrylates, fluoroacrylates, vinyl esters, acrylamides, maleimides, α-olefins, p-alkyl styrenes, alkylvinyl ethers, alkylethylene oxides, triglycerides (e.g. tristearin and pentaerythritol tetrastearate), alkyl phosphazenes and amino acids; polyisocyanates; polyurethanes; and polysiloxanes; as described for example in J. Poly. Sci. 60, 19 (1962), J. Poly. Sci, (Polymer Chemistry) 7, 3053 (1969), 9, 1835, 3349, 3351, 3367, 10, 1657, 3347, 18, 2197, 19, 1871, J. Poly. Sci, Macromol. Rev, 8, 117 (1974), Macromolecules 12, 94 (1979), 13, 12, 15, 18, 2141, 19, 611, JACS 75, 3326 (1953), 76; 6280, Polymer J 17, 991 (1985); and Poly. Sci USSR 21, 241 (1979).

SCC's for use as casting polymers in this invention can be broadly defined as polymers which comprise repeating units of the general formula 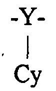 where Y is an organic radical forming part of a polymer backbone and Cy comprises a crystallizable moiety and is preferably present in amount such that the SCC has a heat of fusion of at least 20, preferably at least 40, Joules/gram. The crystallizable moiety may be connected to the polymer backbone directly or through a divalent organic or inorganic radical, e.g. an ester, amide, carbonyl, carboxy, amino, hydrocarbon (for example phenylene), ether or thioether link, or through an ionic salt linkage (for example a carboxyalkyl ammonium, sulfonium or phosphonium ion pair). The radical Cy may be aliphatic or aromatic, for example alkyl of at least 10 carbons, fluoralkyl of at least 6 carbons or p-alkyl styrene wherein the alkyl contains 6 to 24 carbons. The SCC may contain two or more different repeating units of this general formula. The SCC may also contain other repeating units, but the amount of such other units is preferably such that the total weight of the crystallizable moieties is at least twice the weight of the remainder of the polymer.

Preferred SCC's comprise side chains containing in total at least 5 times as many carbon atoms as the polymer backbone, particularly side chains comprising linear polymethylene moieties containing 12 to 50, especially 16 to 22, carbon atoms. The SCC may also contain units derived from one or more other monomers, e.g. other alkyl acrylates, methacrylates (e.g. glycidyl methacrylate), acrylamides and methacrylamides; acrylic and methacrylic acids; acrylamide; methacrylamide; maleic anhydride; 2-isocyanatoethyl methacrylate; comonomers containing amine groups, styrene; vinyl acetate; monoacrylic functional polystyrene; ethyl vinyl ether; vinyl chloride; hydroxyalkyl acrylates and methacrylates; alkoxyalkyl acrylates and methacrylates; and derivatives of polyethylene glycol with molecular weights from 50 to 5,000. Such other monomers are generally present in total amount less than 50%, particularly less than 35%, especially less than 25%, e.g. 0 to 15%. They may be added to modify the melting point, modulus, MVTR, or other physical properties of the SCC, or to provide sites for crosslinking.

One example of an SCC is an SCC consisting essentially of units derived from 0-100% hexadecyl acrylate (C16A), 0-100% octadecyl acrylate (C18A) and 0-20% of one or more of acrylic acid, methacrylic acid, itaconic acid, maleic anhydride or a similar monomer providing a cure site on the copolymer.

The SCC may also be reacted with one or more other materials which produce a strengthened material, for example a multifunctional agent such as amine-terminated propylene oxide, polyethylene oxide, polytetrahydrofuran and polybutadienes containing hydroxyl groups, di-, tri- or multifunctional acrylic or methacrylic esters, vinyl ethers, esters and amides, isocyanates, aldehydes and epoxies.

The SCC may also be reacted with another polymer, or formed on another polymer, so that it is present as one of the blocks in a block copolymer. Such block copolymers are described for example in WO-A-93/07194 (International Patent Application No. PCT/US92/08508 filed October 6, 1992). An SCC polymer as described above can provide the hard block, with the soft block being provided by an amorphous block having a Tg less than (Tₘ - 10)°C or a crystalline block (which may also be an SCC block) having a melting point Tₘₛ less than (Tₘ - 10)°C. Alternatively, an SCC polymer as described above can provide the soft block, with the hard block being provided by an amorphous block having a Tg greater than (Tₘ + 10)°C or a crystalline block having a melting point Tₘₕ greater than (Tₘ + 10)°C. Suitable amorphous blocks are well known in the art and include polyethers, polystyrene, polyacrylates, polyesters and polyurethanes.

The molecular weight of the casting polymer is generally more than 5,000, and when it is the sole polymeric ingredient of the casting composition is preferably at least 50,000, particularly at least 100,000. When the casting polymer is a block copolymer containing SCC blocks, its molecular weight is preferably more than 25,000, especially more than 75,000, with the molecular weight of each SCC block preferably being 2,500 to 20,000.

The casting polymer can be crosslinked by radiation or chemical crosslinking methods known to those skilled in the art. Radiation crosslinking can be effected, for example, by an electron beam or Cobalt 60 radiation. Chemical crosslinking can be effected, for example, by means of peroxides or silanes, by ionic crosslinking, or with the aid of multifunctional agents as described above.

### Casting Compositions

The casting composition can contain, in addition to the casting polymer of melting point Tₘ, one or more additional polymers and/or one or more non-polymeric ingredients, e.g. inorganic fillers, plasticizers, antioxidants, processing aids, and pigments, for example carbon black, graphite, glass fibers, Kevlar fibers, silica, talc and calcium carbonate.

Conventional SCC polymers tend to have poor physical properties, and this limits their value as casting polymers. SCC block copolymers and strengthened or crosslinked SCC polymer have better physical properties, but require additional preparative steps. We have discovered that improved physical properties can be obtained by blending the SCC polymer with an appropriate additional polymer.

We have obtained particularly good results by making use of an SCC polymer which contains at least 30% of units derivable from at least one n-alkyl acrylate or methacrylate in which the n-alkyl group contains 14 to 50, preferably 18 to 30, especially 20 to 24, carbon atoms and 7 to 65%, preferably 9 to 60%, especially 18 to 42%, of units derived from a high Tg monomer, i.e. a monomer which, if homopolymerized, gives a polymer having a Tg which is at least (Tₘ + 10)□C, preferably at least (Tₘ + 20)□C, especially at least 80□C, but which is preferably not more than 120□C particularly not more than 100□C. The preferred high Tg monomer is styrene; other high Tg monomers include α-methyl styrene and other aromatic compounds containing vinyl groups, methyl methacrylate, ethyl methacrylate, t-butyl acrylate and phenoxy ethyl acrylate. The SCC can also contain up to 15%, preferably up to 10%, e.g. 2 to 7%, of units derived from other monomers, preferably acrylic acid. The molecular weight of the polymer is preferably 25,000 to 500,000, particularly 50,000 to 200,000. Such an SCC polymer can have a surprisingly high modulus, often more than 40,000 psi, (28,000 kg/cm²), a sharp melting point, low melt viscosity and good recrystallization kinetics.

The properties of such an SCC polymer can be yet further improved, in particular its impact strength can be increased, by mixing with it a random ethylene copolymer which comprises at least 30% of units derived from ethylene and 7 to 70% of units derived from at least one ethylenically unsaturated monomer containing at least one polar group. The compatibility of these polymers is highly surprising. The ethylene copolymer is preferably an ethylene/vinyl acetate copolymer containing 16 to 60%, particularly 26 to 42% of units derived from vinyl acetate, and preferably also containing 2 to 10% of units derived from acrylic acid. The ratio of the SCC polymer to the ethylene polymer is preferably from 0.25 to 5, particularly 0.5 to 4.

The blends just described are particularly valuable examples of blends which comprise an SCC polymer and a second polymer which improves the physical properties of the SCC polymer. Thus we have discovered that improved physical properties can often be obtained by blending an SCC polymer with at least one compatible olefin polymer, especially an ethylene/vinyl acetate or ethylene/vinyl acetate/acrylic acid copolymer. Good results are obtained when the SCC polymer contains units derived from acrylic or methacrylic acid, preferably in amount 2 to 10%, particularly 3 to 7%, and has a molecular weight of less than 15,000, preferably less than 10,000, and the EVA polymer contains 25 to 50%, preferably 28 to 35%, of units derived from vinyl acetate; particularly good results are obtained when the EVA polymer also contains units derived from acrylic or methacrylic acid, e.g. in amount 2 to 10%. Other olefin polymers which can give improved physical properties include other ethylene copolymers, including ethylene/vinyl chloride, ethylene/acrylic acid, ethylene/ethyl acrylate, ethylene/butyl acrylate, and ethylene/methyl acrylate copolymers. The mixture can also contain an ionic component, e.g. a zinc acrylic acid complex.

Another way in which the physical properties of SCC polymers can be improved is by blending them with polycaprolactone (PCL) or another cyclic ester polymer as disclosed in U.S. Patent No. 3,692,023. Some, but not all, PCLs and related polyesters have melting points which are too high to permit their use in casts which are heated after application to the patient, and also take a long time to crystallize after cooling below Tₘ. However, we have found that mixtures of SCC polymers and PCL can have a Tₘ substantially below that of the PCL on its own, without undue broadening of the melting range. Preferred SCC polymers for this purpose comprise units derived from C16A and/or C18A, and optionally units derivable from one or more other monomers selected from n-alkyl acrylates and methacrylates in which the alkyl group contains less than 18 carbons, acrylic acid, methacrylic acid, acrylamide, and methacrylamide. Particularly preferred SCC polymers are C16A/C18A/AA copolymers.

As briefly indicated above, the casting compositions can contain a wide variety of fillers and other non-polymeric ingredients. However, we have found that certain fillers give better results than others, in particular in improving the modulus and/or elongation of the composition, or the balance between modulus and elongation, particularly with casting compositions containing an ethylene/vinyl acetate copolymer in addition to an SCC casting polymer. We have obtained particularly good results through the use of calcium carbonate (both untreated and surface-treated), wollastonite (acicular calcium silicate) and talc (magnesium silicate).

### Supports

The novel casts comprise a fabric support for the polymer, particularly a woven, knitted, braided or non-woven fabric. The support preferably has, in the cast before it is molded, an open structure which assists in the heat transfer between different parts of the cast, and which, in the finished cast, remains sufficiently porous to ensure that the support has a high moisture vapor transmission rate (MVTR) and thus does not prevent the substrate from "breathing". When a support in a heat-recoverable cast has an open structure, the size of the apertures therein will decrease during recovery. Recovery will also make the support more rigid. Thus the cross-sectional area of the components of the support will normally increase during recovery, and previously separated components will come closer together and be bonded together by the casting polymer.

The support is elastically deformable. In a fabric, elasticity can result from the construction of the fabric and/or the inherent elasticity of the fibers therein. For example, a support which can be used in this invention can be a knitted fabric prepared entirely from a glass fiber yarn. However, preferred supports are fabrics, particularly knitted fabrics, in which at least one of the yarns is composed of an elastomeric material. The fabric may be isotropic or have elasticity or other properties which vary directionally. Preferably the support can be stretched elastically in at least one direction by at least 25%, preferably at least 50%, e.g. 50 to 125%, based on the corresponding dimension of the support in its unstretched state. For example, the support can be a knitted, woven or braided material which comprises an elastomeric yarn, e.g. a segmented polyurethane yarn, or a natural or synthetic rubber yarn, preferably with a non-elastomeric yarn, e.g. a glass fiber, graphite, polyamide or polyarylene yarn, which is selected to give the cast desired strength or other physical properties. Such support materials are described for example in US Patent No. 4,668,563 (Buese), and are available commercially, for example nylon/segmented polyurethane fabrics and glass fiber/segmented polyurethane fabrics. The power of the fabric is preferably 0.1 to 2.0 lb/inch (18 to 360 g/cm). The support preferably has, at least after stretching, an open structure, so that the coated support (i.e. the cast) can also have an open structure, as further described elsewhere in this specification.

### Casts

It is desirable that the recovery forces should be sufficient to ensure adequate conformance of the cast to the substrate, but not so high as to damage the substrate, or cause pain, or reduce blood flow. The power of the cast, in the direction of recovery, is, therefore, preferably 18 to 360 g/cm (0.1 to 2.0 lb/inch) . Similarly, when the molding of the cast is effected at least in part by externally applied forces, e.g. from the orthopedist's hands, the cast should be easily molded but remain cohesive. The strength of the cast, at the time of molding, is therefore, preferably 9 to 900 (0.05 to 5), particularly 18 to 540 (0.1 to 3) especially 45 to 360 (0.25 to 2), g/cm,

Part or all of the interior surface of the casts of the invention can be coated with an adhesive. The adhesive can be for example a temperature-activated adhesive as described in WO-A-90/13420 (International Application No. PCT US90/02223) or a pressure-sensitive adhesive (PSA), for example a PSA as disclosed in WO-A-92/13901 (International Patent Application No. PCT US92/01153), particularly a PSA containing an SCC polymer additive which reduces the adhesive strength of the PSA when the PSA is warmed. The use of such an adhesive is often particularly desirable when the cast surrounds a finger and needs to resist removal by the patient (such casts are often referred to as "Mallet Finger" casts) by laminating such a sheet to a support.

When the cast comprises a sheet of the casting composition or is made by laminating such a sheet to a support, the sheet is generally 1 to 5 mm thick, with smaller thicknesses, e.g. less than 2 mm, being preferred for smaller casts, e.g. for fingers, toes and hands.

We have found that the casts are much easier to apply, without overheating which can cause distress to the patient or the orthopedist, if they have, before molding, an open structure which permits rapid and uniform heating of the casting composition, especially when using a hot air gun to heat the cast. The open structure is preferably such that over substantial areas of the cast, particularly over the whole of the casting polymer, heating the cast with a hot air gun causes a difference in temperature, between the inside and the outside of the cast at any particular point, of not more than 15°C, particularly not more than 10°C, especially not more than 5°C. We have obtained excellent results with casts which, before molding have an air flow permeability (AFP) of at least 10%, particularly at least 20%.

When an unused cast is molded into its finished state, any apertures therein will become smaller (or disappear completely) as a result of recovery and/or thermoplastic flow of the casting polymer. However, it is very desirable that the finished cast should retain a sufficiently open structure to allow the substrate to "breathe", i.e. should have a good moisture vapor transmission rate (MVTR). It is, therefore, preferred that the apertures in the unused cast should remain sufficiently large in the finished cast to provide this benefit. We have found that the AFP of the finished cast is preferably at least 1%, more preferably at least 2%, particularly at least 5%, especially at least 8%,. On the other hand, the larger the size and the number of apertures in the finished cast, the weaker and less water-resistant it will be. Preferably, therefore, the AFP of the finished cast is less than 25%, more preferably less than 20%, particularly less than 15%, especially less than 10%. The preferred upper values for AFP in the unused cast are somewhat higher (because the apertures become smaller during molding of the cast). The AFP of the unused cast is preferably less than 45%, more preferably less than 35%, particularly less than 30%, especially less than 25%.

The size and number of the apertures in the unused and finished cast are important in determining the ease with which the cast can be applied and its performance. We have obtained excellent results using apertures having an area from 0.01 to 0.08 cm², preferably 0.02 to 0.06 cm², e.g. about 0.04 cm².

When a cast is prepared by laminating together two or more porous components, e.g. by wrapping 2, 3 or 4 thicknesses of a porous sheet material, the performance of the cast reflects the porosity of the individual components so that when a cast is prepared in this way, the sheet material preferably has an AFP of at least 15%, particularly at least 25%, but less than 60%, particularly less than 40%, especially less than 30%.

It is desirable that the cast should become relatively inflexible in a short period after molding is complete. Preferably, therefore, the casting composition, as it cools, changes from a moldable composition to an inflexible composition over a temperature range of less than 10°C, particularly less than 5°C, and preferably does so in less than 2 minutes, particularly less than 1 minute. The casting polymer need not recover all of its crystallinity in order for the casting composition to become substantially inflexible. In this specification, the polymer can be regarded as having cooled to a crystalline inflexible material if its crystallinity is at least 0.5 X, where X is the crystallinity of the polymer after it has been cooled from above Tₘ to (Tₘ-10)°C and maintained at (Tₘ-10)°C for four crystallization half lives.

After it has been molded and has then cooled, the cast should be strong and relatively inflexible. The cast preferably has, therefore, a strength of more than 5, particularly more than 10, especially more than 15, pounds per inch. The pressure exerted by the finished cast on the substrate should be low enough to prevent damage thereto, either immediately or while the cast is being worn (e.g. swelling, ulcers, tissue neurosis and decreased blood flow), and may be for example 10-40 mm Hg above atmospheric.

The casts of the invention can take many forms, including (1) non-recoverable tapes and sheets which are heated and molded in place by the orthopedist; (2) elastic tapes and sheets which are not recoverable but which are heated and then stretched by the orthopedist as they are applied to a substrate; (3) recoverable tapes and sheets which are flexible enough to be wrapped around a substrate while they are cold and are then heated and recovered to form a cast; and (4) relatively rigid heat-recoverable casts which are in approximately the desired final shape, but sufficiently oversize to allow the cast to be placed around the substrate, and which are placed around the substrate and then heated to recover onto the substrate. To assist in fitting such a preshaped cast to the substrate, and/or to assist in its later removal from the substrate, the cast may comprise (1) a first component which (i) has an open cross-section and (ii) comprises the casting polymer, and (2) a second component which (i) forms a closed cross-section with the first component and (ii) is elastomeric when the article is in its first (recoverable) configuration and/or when the article is in its second (fitted) configuration. The second component can be secured to the first component after the first component has been formed, or a casting polymer can be applied to part only of an elastomeric support. Alternatively or additionally, the cast can be in the form of two or more parts which can be fitted together around the substrate and secured together before heating begins. One or more of the component parts can carry means for securing the parts together, preferably means which permit the size of the cast to be adjusted, e.g. hook-and-loop closures of the kind sold under the trademark "Velcro".

Pre-shaped casts may have a wide variety of shapes, including for example a cylinder for use as a finger splint, a bent cylinder to surround an ankle, knee or elbow, or a glove to surround part or all of a hand.

### Preparation of Casting Compositions and Casts

The casting compositions used in this invention can be prepared by mixing procedures well known in the art, and can be formed or applied to supports by procedures well known in the art, e.g. as solutions in organic solvents which are removed by drying, or as molten compositions, e.g. by melt extrusion onto the support or by hot lamination. Where the support is an elastic support, the casting composition is preferably applied to the non-deformed support, and the composite then stretched e.g. over a mandrel or other form, while the casting composition is at a temperature above Tₘ, followed by cooling with the support in its stretched condition. However, the casting composition can also be applied to the stretched support and solidified on it. After the composition has been applied to a support, it may be desirable to heat it, at a temperature well above Tₘ, preferably with pressure, to ensure that it fully penetrates the support. It may also be desirable to blow air through the casting composition to ensure that the open structure of a porous support is not filled up by the casting polymer.

### EXAMPLES

This invention is illustrated in the following Examples, in which the following abbreviations are used to refer to the compounds and materials set out in parentheses after the abbreviation:
AA (acrylic acid), C4A (butyl acrylate), C14A (tetradecyl acrylate), C16A (hexadecyl acrylate), C18A (octadecyl acrylate), C22A (docosanyl acrylate), MMA (methyl methacrylate), C4MA (butyl methacrylate), C18MA (octadecyl methacrylate), GMA (glycidol methacrylate), ETGMA (ethyl triglycidol methacrylate), STY (styrene), C12SH (dodecyl mercaptan), XAMA (a polyfunctional crosslinking agent sold by Hoechst Celanese under the trade name Xama 2), ESP570 (t-amylperoxy 2-ethyl hexanoate), ESP 5100 (t-amylperoxy benzoate), JT and JD (polyfunctional amines sold by Texaco under the trade names Jeffamine T-3000 and Jeffamine D-4000, respectively), PCL (polycaprolactone sold by Polymer Sciences under the trade name PCL640),.ELV40, ELV150, ELV240, ELV250, ELY4260 and ELV4320 (different grades of ethylene/vinyl acetate copolymers sold by E. I. duPont de Nemours under the trade names Elvax 40, Elvax 150, Elvax 246, Elvax 250, Elvax 4260 and Elvax 4320 respectively), EY901 and EY902 (different grades of ethylene/vinyl acetate copolymers sold by Quantum Chemical Co. Inc. under the trade names Vynathene EY901-25 and EY 902-35 respectively), HEP (heptane), TOL (toluene), NL Knit (a knitted fabric which contains about 85% polyhexamethylene adipamide (nylon) fibers and about 15% segmented polyurethane (Lycra) fibers, has a thickness of about 0.023 cm (0.015 inch) and a weight of about 95 g/m², has a linear stress strain curve at extensions up to about 126%, and exhibits a power of about 135 g/cm 0.75 lb/inch), GLF (0.75) and GLF (2.0) (E-type non-woven random glass fiber mats which have weights of about 0.022, 0.044 and 0.06 g/cm² (0.75, 1.5 and 2.0 oz/sq ft) respectively, sold by Tap Plastics), GRAPH (a non-woven graphite mat sold under the trade name Panex CPF by Stackpole), GL Knit (a warp knitted fabric which contains glass fiber yarn and an elastic yarn, which has a weight of about 530 g/m², and which is available from Carolina Narrow Fabric Co., Winston-Salem, North Carolina, USA), DAB9 (calcium carbonate sold by United Minerals Co. under the trade name DAB9), W-30 (Wollastonite sold by R. T. Vanderbilt Co. under the trade name W-30), and Nytal (talc sold by R. T. Vanderbilt under the trade name Nytal 400).

### SCC Polymers

The first step in the Examples was to make SCC polymers S1 to S23, using the monomers and amounts thereof specified in Table 1 below. The monomers were placed in a vessel with a suitable solvent, and optionally with azobisisobutyronitrile (AIBN) as an initiator and/or C12SH as a chain transfer agent. These ingredients were heated under nitrogen, with stirring, at an elevated temperature, e.g. 60-70°C, for an appropriate time, e.g. 12-20 hours. After cooling, the SCC polymer was precipitated by pouring the reaction mixture into ethanol. For example, in preparing polymer S1, the monomers and 0.5 g AIBN were dissolved in 150 mL toluene, and heated at 70°C for 12 hours. In preparing polymers S3-S11, the monomers were dissolved in sufficient toluene to give a reaction mixture containing 42% monomers, and the reaction mixture was heated at 60°C for 16 hours. In preparing polymer S12, the monomers, 0.05 g C12SH, and 3.5 g AIBN were dissolved in a mixture of 1400 mL heptane and 80 mL ethyl acetate, and the reaction mixture was heated at 60°C for 20 hours. In preparing polymer S20, 1700 g of a mixture of C22A, STY, AA and C12SH (ratio 60/37/3/0.19) was maintained at 105°C while adding a blend of the same mixture (6800 g) and ESP570 (42.5 g) over 90 mins; ESP 5100 (42.5 g) was then added over 45 mins, while raising the temperature to 140°C; and this temperature was maintained for a further 230 minutes. Polymers S21-23 were made in a similar way.

### Examples 1-15

In Examples 1 to 15, the next step was to prepare the fabric support, using the fabric designated in Table 2 and as further described below. The fabric support was then contacted with a composition containing the ingredients and amounts thereof set out in Table 2 and processed as further described below.

Example 1. The NL Knit fabric was sewn into a tube of diameter about 1.6 cm (0.63 inch) having maximum elasticity in the radial direction. The fabric tube was fitted over a mandrel of diameter 1.9 cm (0.75 inch), and was then coated with the SCC polymer composition, which also contained 25 parts of heptane as solvent. After the solvent had been removed by heating, the weight of polymer deposited on the fabric was about 1 g. per inch of the tube measured in the axial direction. The coated tube was removed from the mandrel and retained its expanded shape. It could be easily deformed, for example by pressing into a flat sleeve. Two 7.6 cm (3 inch) lengths were cut from the tube. One length was placed over a finger, and then heated with a hair dryer, causing it to shrink and fit snugly over the finger. The second length was placed over the first, and likewise shrunk. After 2 minutes, the two lengths had hardened into a composite cast which immobilized the finger but did not restrict its blood flow. The cast was later reheated, when it became soft and flexible, so that it could be removed, or the finger and cast could be repositioned into a different shape, e.g. a bend; after two minutes the cast had again cooled and hardened, immobilizing the finger in its new shape.

Example 2. A strip of the NL Knit fabric was stretched about 50% and coated with the SCC polymer composition, heated at 90°C for 2 hours, and cooled. A first sample of the resulting flexible coated fabric was wrapped around a finger and then heated; the fabric softened and shrank snugly around the finger, and on cooling formed a rigid cast, with the overlapping parts of the fabric adhering to each other, which immobilized the finger but did not restrict its blood flow. A second sample was heated and then wrapped around a finger, using light tension; on cooling, the fabric formed a rigid cast which immobilized the finger but did not restrict its blood flow. A third sample was used in the same way as the first, but greater tension was used when wrapping; on cooling, the resulting cast was uncomfortably tight, and restricted blood flow.

Example 3. In each of Examples 3A, 3B and 3C, the dry SCC polymer was placed on top of the GLF fabric, and the fabric and polymer were heated between sheets of siliconized release paper in a press at 100°C and 14 kg/cm² (100 psi). The ratio by weight of fabric to polymer was about 1.5. The resulting composites were stiff at temperatures below about 39°C, but flexible at temperatures above about 42°C.

Example 4. A sample of the composite of Example 3C, about 10 x 10 cm (4 x 4 inch), was laminated between two sheets of a polyester thermoplastic elastomer ("Hytrel" 4056) about 0.0025 cm (0.001 inch) thick. The laminate was cut into the shape of a nose splint, warmed with a hair dryer to make it flexible, and formed over a nose. After cooling, it provided a rigid nose splint.

Example 5. Another, larger, sample of the composite of Example 3C was laminated between 0.0044 cm (0.00175 inch) thick polyester film ("Medifilm 325" from Bertek) and a 0.0025 cm (0.001 inch) thick film of polymer S12, which is a temperature-responsive adhesive. The resulting composite was rigid and non-tacky at room temperature. It was cut into the shape of a Vilinus nose splint, warmed with a hair dryer to make it flexible and to make the adhesive tacky, and then formed over the nose and forehead of a person. After cooling, it provided a rigid splint bonded to the nose and forehead.

Example 6A. The NL Knit fabric was sewn into a tube about 91 cm (36 inch) long and about 1.4 cm (0.55 inch) in diameter. The outside of the tube was coated with the SCC polymer solution, which also contained toluene (30 parts), and then dried at 135°C for 3 hours. The coating weight was about 95 g/m². The coated tube was cut into 10 cm (4 inch) lengths which were heated with a hot air gun to make them flexible and then expanded by 100% over a mandrel, and cooled on the mandrel. A first expanded length was placed over a finger and then heated with a hair dryer, causing it to shrink and fit snugly over the finger. A second expanded length was placed over the first and likewise shrunk, forming a composite cast which was significantly stronger than the cast produced in Example 1. A 2.5 cm (1 inch) length of the coated tube was cut lengthwise so that its mechanical properties could be measured. At 25°C, the fabric was inelastic and had a strength of about 1430 g/cm (8 lb/inch). At 50°C, the fabric had a power of about 204 g (0.45 lb), was reversibly elastic, and exhibited a linear stress/strain curve of about 100% elongation.

Example 6B. The NL Knit fabric, in flat sheet form, was coated with the SCC polymer solution and then dried at 135°C for 3 hours. A 21.5 x 28 cm (8.5 x 11 inch) of the coated fabric was folded in half, and then cut and sewn so that it comprised a first tube about 10 cm (4 inch) long with a diameter of about 6.3 cm (2.5 inch), and a second tube about 6.3 cm (2.5 inch) long with a diameter of about 1.9 cm (0.75 inch) which communicates with the first tube and is at an angle of about 60° to it. This structure can be heated, expanded while hot, and cooled in the expanded state. The resulting structure is a cast which can be placed over a hand to cover the wrist, part of the palm of the hand and the thumb, and which, when heated with a hair dryer, shrinks and forms, after cooling, a rigid cast which immobilizes the thumb.

Example 7. One of the expanded lengths produced in Example 6 was lightly coated on the inside with a 30% solution of the S12 SCC polymer, which was a temperature-responsive adhesive. After it had been dried, the adhesive was non-tacky at room temperature. The adhesive-coated length was placed over a finger and then heated with a hair dryer, causing it to shrink and to form, after cooling, a rigid cast bonded to the finger.

Example 8. The graphite fabric was coated with the SCC composition, which also contained toluene (30 parts) and then dried at 140°C for 2 hours. The coating weight was about 201 g/m² (0.132 g/inch²). A coating about 0.0025 cm (0.001 inch) thick of the S12 SCC polymer adhesive was formed on a polyester thermoplastic elastomer ("Hytrel" 4056) film 0.0045 cm (0.00175 inch) thick. The coated fabric and the coated film were laminated together, with the SCC polymers in contact. To the other side of the graphite fabric was bonded a 0.0051 cm (0.002 inch) thick film of an acrylic pressure-sensitive adhesive sold by Monsanto Chemical as a solution under the trade name Gelva 737. The resultant composite was rigid at room temperature; when warmed with a hair dryer, became flexible and could be conformed around a substrate; and after cooling provided a rigid protective shell bonded to the substrate. For example, a 7.5 x 7.5 cm (3 x 3 inch) sample of the composite was used in this way to provide a rigid molded protective cast bonded to a person's heel.

Example 9. The blend of the S1 SCC polymer and the PCL was made by melt mixing at 100°C. The blend had a Tₘ of 44°C and a recrystallization temperature of 34°C; a sample of it 7.6 x 10.1 x 0.076 cm (3 x 4 x 0.03 inch) could be warmed with a hair dryer to a temperature at which it was flexible, could be wrapped around a finger, and allowed to cool, when it formed a rigid cast around the finger. The PCL on its own had a Tₘ of 60°C and a recrystallization temperature of 23°C; a similar sample of PCL on its own, when heated to a temperature at which it was flexible, could not be wrapped around a finger because the finger could not tolerate the heat.

Examples 10A and 10B. A sample of S3 SCC polymer was heated to 100°C for 5 minutes and placed on a metal block maintained at 32°C. The SCC polymer began to harden after 20 seconds and was fully hardened after 60 seconds. The PCL polymer, similarly treated, began to harden after 90 seconds, and was not fully hardened until after 4 minutes.

Example 11. The blend of S1 SCC polymer and the PCL was made by melt mixing. The blend has a Tₘ of about 42°C. The blend was hot melt coated onto the NL Knit fabric, and cooled. A sample about 7.5 x 10 cm (3 x 4 inch) was cut from the coated fabric; warmed in water at 60°C, thus rendering it flexible; and wrapped around a finger. After cooling for 3 minutes, it provided a rigid splint around the finger.

Example 12. The coated fabric of Example 3B was laminated between two sheets of polyurethane film 0.0044 cm (0.00175 inch) thick. ("Tuftane" 4056, sold by Lord Corporation, Erie, Pennsylvania), each sheet being coated on its inner surface with a layer of an acrylic pressure-sensitive adhesive (Gelva 737). The resulting laminate was rigid at room temperature. However, by exerting sufficient force, it could be wrapped around a 0.63 cm (0.25 inch) mandrel, thus fracturing the rigid parts of the laminate, and making it possible to wrap the laminate around a person's wrist. The wrapped laminate was heated with a hair dryer, thus softening the laminate, and then allowed to cool, when it formed a rigid splint around the wrist.

Example 13. The solution of the SCC polymer (5 parts of one of the polymers S3 to S11dissolved in 5 parts of toluene) was coated onto the NL Knit fabric so that the weight of the solution was 40-60% of the weight of the uncoated fabric and heated at 135°C for 4 hours. The cooled composites were inelastic at room temperature, but were flexible and could be stretched by up to 50% when heated to 50°C. When the stretched composite was cooled to room temperature, it again became inelastic. For example, 2.5 x 10.2 cm (1 x 4 inch) strips were stretched about 50% at 50°C; cooled in the stretched state; loosely wrapped around a 1.25 cm (0.5 inch) mandrel; and heated to 50°C. The strips recovered, providing a rigid, protective coating conforming to the mandrel, with overlapping sections of the strips adhered to each other.

Example 14. Several 7.6 cm (3 inch) lengths were cut from the expanded tube of coated NL Knit fabric prepared in Example 6, and a layer of a medical grade pressure-sensitive adhesive in the form of a transfer tape ("Avery I-780" available from Avery Dennison) was applied to the outside of the lengths. Strips 2.5 cm (1 inch) wide were cut lengthwise from the lengths. These strips recovered when heated, and became inelastic when cooled. For example, one strip was applied to loose skin on a person's bicep. When the strip was heated with a hair dryer, it contracted, pulling under the strip together and uniformly stretching the surrounding skin. Thus a strip of this type can be applied over a wound or incision and, when desired, heated to close and protect the wound or incision. A strip of this type can also be used to stretch an area of skin, e.g. around a burn.

Example 15. The blend of S13 SCC polymer and Elvax 240 was made by melt mixing. The polymers appeared to be completely compatible in the melt. A sample of the blend was melt pressed to a thickness of about 0.0127 cm (0.005 inch), laminated to the NL Knit fabric at 100°C, and allowed to cool. The laminate was heated to 60°C, stretched 100%, and cooled in the stretched state. When reheated, the stretched fabric contracted, and on cooling became rigid.

In Examples 16 and 17, the composition designated in Table 2 was prepared and used in the way described below.

Example 16. The specified blends of SCC polymer and Elvax were prepared by melt mixing. In each of Examples 16A, B and D, the polymers appeared to be completely compatible, and the blend could be melted easily and molded on human skin to provide a protective coating. However, in Example 16C, the polymers were not compatible, and the blend did not soften sufficiently to be useful as an orthopedic device at temperatures which could be tolerated by human skin.

Example 17. The specified blends of S18 SCC polymer and Jeffamine in Examples 17A, B, C and D were made by melt mixing. 50g samples of the S18 SCC polymer on its own (Example 17E) and of the blends (Examples 17A, B, C and D) were melt processed into plaques and heated under pressure for 4 hours at 150°C. At room temperature, Sample E was opaque and very brittle. Samples A, B, C and D were strong and flexible at room temperature, and when heated to 40°C became soft and conformal. Sample C had a desirable low melt modulus and high melt elongation. Sample D had a relatively high melt modulus and relatively poor melt elongation. Sample B was transparent, so that a wound covered by it could be inspected without removing the sample.

### Examples 18-24

In Examples 18-34, the ingredients and amounts thereof set out in Table 2 were made by melt mixing in a Brabender. The modulus and elongation of the blends in Examples 19-34 were measured.

In Example 22, the polymeric composition (S20 + EY901) was hot melt coated as a layer about 0.01 inch (0.025 cm) thick onto the GL Knit fabric. The coated fabric was maintained at 100°C for 4 hours and cooled. The resulting composite was about 0.07 inch (0.18 cm) thick. The composite was then heated with a hot air gun to soften the polymeric composition, stretched 100%, and cooled in the stretched state. A sample of the cooled fabric, 12 by 48 inch (30.5 by 122 cm) (stretched lengthwise) was wrapped 3 times around a metal tube of 12.7 cm diameter and the exterior wrapped end was bonded to the layer below with a polyamide hot melt adhesive. The wrapped fabric was heated to recover it onto the tube, and after cooling, the expanded cast was pulled off the tube. The cast was placed over a person's wrist and forearm, and heated with a hair dryer. It recovered fully within 100 seconds and after cooling provided a rigid cast.

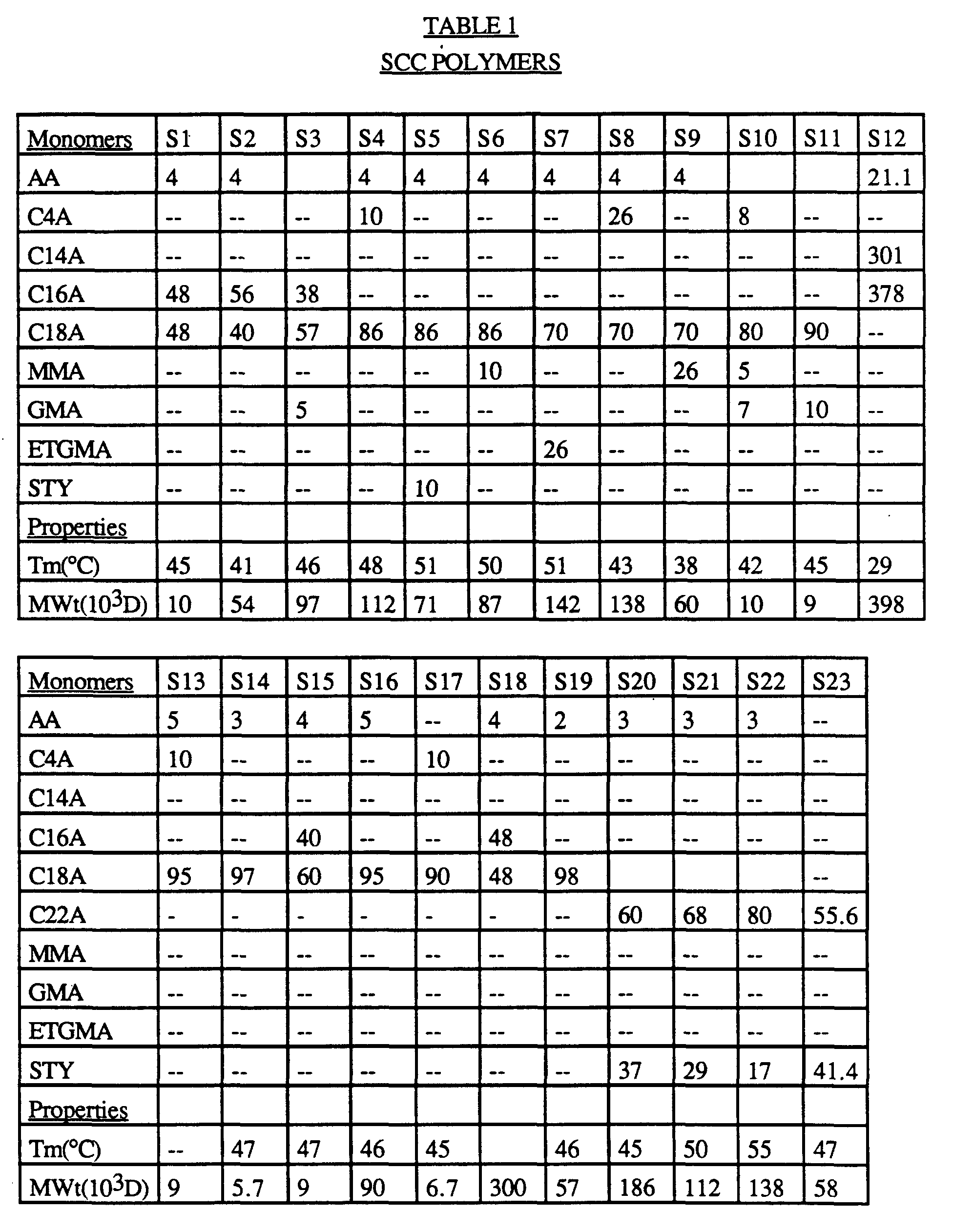

**TABLE 2**

| COMPOSITIONS AND FABRICS | | | | | |
|---|---|---|---|---|---|
| Ex. | COMPOSITION | | Modulus | Elongation | FABRIC |
| No. | SCC | Other | psi | % | |
| 1,2 | S1 (25) | XAMA (0.125) | - | - | NL Knit |
| 3A | S2 (100) | - | - | - | GLF (0.75) |
| 3B | S2 (100) | - | - | - | GLF (1.5) |
| 3C,4,5 | S2 (100) | - | - | - | GLF (2.0) |
| 6 | S3 (25) | JT (5) | - | - | NL Knit |
| 7 | S12 (100) | - | - | - | from Example 6 |
| 8 | S3 (25) | JT (5) | - | - | GRAPH |
| 9 | S1 (5) | PCL (5) | - | - | - |
| 10A | S3 (100) | - | - | - | - |
| 10B | S3 (100) | PCL | - | - | - |
| 11 | S1 (6) | ELV 240 (6) | - | - | NL Knit |
| 12 | S2 (100) | - | - | - | from Example 3B |
| 13 | S3-S11 (5) | - | - | - | NL Knit |
| 14 | S3 (100) | - | - | - | from Example 6 |
| 15 | S13 (5) | ELV240 (5) | - | - | NL Knit |
| 16A | S14 (5) | ELV240 (5) | - | - | - |
| 16B | S15 (5) | ELV4320 (5) | - | - | - |
| 16C | S16 (5) | ELV150 (5) | - | - | - |
| 16D | S17 (5) | ELV250 (5) | - | - | - |
| 17A | S18 (85) | JT (15) | - | - | - |
| 17B | S18 (80) | JT (20) | - | - | - |
| 17C | S18 (80) | JT (20) | - | - | - |
| 17D | S18 (70) | JT (30) | - | - | - |
| 17E | S18 (100) | - | - | - | - |
| 18 | S19 (50) | ELV4320 (50) | - | - | incompatible |
| 19 | S20 (75) | ELV40 (25) | 20.5 k | 70 | - |
| 20 | S20 (80) | ELV40 (20) | 22.5 k | 38 | - |
| 21 | S20 (75) | ELV4260 (25) | 21.2 k | 51 | - |
| 22 | S20 (75) | EY901 (25) | 33.8 k | 27 | GL Knit |
| 23 | S20 (75) | EY902 (25) | 21.2 k | 21 | - |
| 24 | S21 (75) | ELV4320 (25) | | | |
| 25 | S22 (60) | ELV4320 (40) | 21 k | 25 | - |
| 26 | S22 (70) | ELV4320 (25) | 30k | 3 | - |
| 27 | S20 (100) | - | 48k | 2.5 | - |
| 28 | S20 (80) | EY901 (20) | 34k | 27 | - |
| 29 | S20 (60) | ELV4260 (S20) Nytal (20) | 46.2 k | 24 | - |
| 30 | S20 (54) | ELV4260 (18) DAB9 (28) | 41.5 k | 18 | - |
| 31 | S20 (54) | ELV4260 (18) DAB9 (28) | 30.3 k | 44 | - |
| 32 | S20 (58) | ELV4260 (14) DAB9 (28) | 46 k | 34 | - |
| 33 | S20 (60) | ELV4260 (20) DAB9 (20) | 37.4k | 37 | - |
| 34 | S20 (60) | ELV40 (20) DAB9 (20) | 34.6 k | 37 | - |

Example 25. An expanded cast made by the procedure of Example 24 was slit lengthwise. It opened up to a C-shaped cross-section. Opposite edges of a strip of NL Knit fabric about 1.5 inch (3.8 cm) wide were sewn to the slit edges, securing them together and reclosing the tube. The resulting cast was placed over a person's wrist and forearm, and heated with a hair dryer. It recovered, and, after cooling, provided a cast which was sufficiently rigid to prevent movement, but which could expand or contract to accommodate swelling or shrinkage of the wrist or forearm and which could be removed.

Example 26. The procedure of Example 25 was followed except that, instead of the fabric strip, two halves of a zipper were secured to the slit edges of the cast so that the cast could be secured in place by closing the zipper.

Example 27. The procedure of Example 25 was followed except that, instead of the fabric strip, matching parts of a Velcro closure were secured to the inner and outer surfaces, respectively, adjacent the cut edges, so that the cast could be secured in place by pressing together the matching parts.

Example 28. This example shows the advantages of a cast having an open structure. In each of the tests reported below, a flat sample of an unused cast was placed on a metal screen below the tip of a hot air gun at a distance of about 3 inch (7.6 cm) for samples 1, 2 and 3, and about 5 inch (12.7 cm) for samples 4, 5 and 6. Thermocouples were placed on the top and bottom of the sample, and recorded the temperature at 15 second intervals as the sample was heated by a constant flow of hot air from the gun. The various samples used were:
1. A sheet of PCL 0.03 inch (0.076 cm) thick, without apertures.
2. A composite about 0.048 inch (0.12 cm) thick obtained by melt laminating a sheet of PCL 0.03 inch (0.076 cm) thick, without apertures, and a knitted fiberglass tape.
3. A composite about 0.06 inch (0.15 cm) thick obtained by laminating two layers of composite 2 and expanding the laminate 100%; this composite was highly porous.
4. A sheet of the S20 SCC polymer about 0.043 inch (0.11 cm) thick, without apertures.
5. The expanded composite made in Example 22, which was highly porous.
6. The composite made in Example 22, before it was expanded.

The results obtained are set out in Table 3 below, and clearly show the benefits of the open structure in samples 3 and 5, as compared to the non-porous structures of the other samples.

**TABLE 3**

| Temperature of Sample (°C) After (secs) | | | | | | | |
|---|---|---|---|---|---|---|---|
| | | 0 | 15 | 30 | 45 | 60 | 90 |
| Sample 1 | Top | 27 | 61 | 67 | 62 | 61 | - |
| | Bottom | 28 | 40 | 50 | 61* | 62 | - |
| Sample 2 | Top | 26 | 73 | 78 | 76 | 78 | - |
| | Bottom | 25 | 48 | 54 | 62* | 63 | - |
| Sample 3 | Top | 26 | 70 | 78 | - | - | - |
| | Bottom | 26 | 68* | 79 | - | - | - |
| Sample 4 | Top | 24 | 53 | 60 | 67 | 69 | 66 |
| | Bottom | 23 | 36 | 41 | 47 | 52 | 63* |
| Sample 5 | Top | 23 | 68 | 71 | 71 | - | - |
| | Bottom | 23 | 63* | 70 | 70 | - | - |
| Sample 6 | Top | 24 | 64 | 70 | 71 | 74 | - |
| | Bottom | 25 | 42 | 62* | 72 | 62 | - |

| | | | | | | | |
|---|---|---|---|---|---|---|---|
| * At this time, the cast was recovering and/or moldable | | | | | | | |

## Claims

1. A heat-recoverable cast which comprises
(1) a recoverable fabric support which is in a stretched condition; and
(2) a rigid casting composition which
(a) maintains the support in its stretched condition, and
(b) comprises a casting polymer having a crystalline melting point Tₘ;
whereby the cast
(A) at temperatures below Tₘ, has a first stable configuration,
(B) when heated to a temperature above Tₘ in the absence of restraint, becomes moldable and recovers so that at least one dimension thereof decreases from a first value x to a second value y which is at most 0.95 x, and
(C) when cooled to a temperature below Tₘ after being molded, adopts a second, stable configuration.

2. A cast according to Claim 1 wherein the fabric support is a knitted fabric which comprises an elastomeric yarn and a non-elastomeric yarn.

3. A cast according to Claim 2 wherein the fabric has a power, measured as hereinbefore defined, of 18 to 360 g/cm (0.1 to 2.0 lb/inch).

4. A cast according to any one of the preceding claims, at least a part of the cast having a plurality of apertures through its thickness, each of the apertures having an area of 0.01 to 0.08 cm².

5. A cast according to any one of the preceding claims, at least a part of the cast having an air flow permeability, measured as hereinbefore defined, of 10 to 35%.

6. A cast according to any one of the preceding claims wherein the Tₘ of the casting polymer is 45 to 60°C.

7. A cast according to any one of the preceding claims wherein the casting polymer comprises an SCC polymer.

8. A cast according to any one of claims 1 to 6 wherein the casting polymer comprises polycaprolactone.

9. A method of making an orthopedic cast as defined in any one of Claims 1 to 8, which comprises applying the casting composition, while it is molten, to the fabric support.

10. A method according to Claim 9 which comprises laminating together two or more porous sheet materials.

## Patentansprüche

1. Wärmerückstellbares Gußstück, das
(1) einen rückstellbaren Textilträger, der im gestreckten Zustand vorliegt, und
(2) eine starre Gußzusammensetzung umfasst, die
(a) den Träger in seinem gestreckten Zustand hält und
(b) Gußpolymer mit einem kristallinen Schmelzpunkt Tₘ umfasst,
wobei das Gußstück
(A) bei Temperaturen unter Tₘ eine erste stabile Konfiguration hat,
(B) nach Erwärmung auf eine Temperatur über Tₘ in Abwesenheit von Behinderung formbar wird und sich rückstellt, so daß sich mindestens eine erste Dimension desselben von einem ersten Wert x auf einen zweiten Wert y vermindert, der höchstens 0,95 x ist, und
(C) nach Abkühlung auf eine Temperatur unter Tₘ, nachdem es geformt worden ist, eine zweite stabile Konfiguration annimmt.

2. Gußstück nach Anspruch 1, bei dem der Textilträger gestricktes Textil ist, das elastomeres Garn und nicht-elastomeres Garn umfasst.

3. Gußstück nach Anspruch 2, bei dem das Textil eine wie zuvor hier definiert gemessene Kraft von 18 bis 360 g/cm (0,1 bis 2,0 lb/ Zoll) hat.

4. Gußstück nach einem der vorhergehenden Ansprüche, bei dem mindestens ein Teil des Gußstücks durch seine Dicke hindurch eine Vielzahl von Öffnungen aufweist, wobei jede der Öffnungen eine Fläche von 0,01 bis 0,08 cm² hat.

5. Gußstück nach einem der vorhergehenden Ansprüche, bei dem mindestens ein Teil des Gußstücks eine wie hier zuvor definiert gemessene Luftdurchlässigkeit von 10 bis 35 % hat.

6. Gußstück nach einem der vorhergehenden Ansprüche, bei dem die Tₘ des Gußpolymers 45 bis 60°C ist.

7. Gußstück nach einem der vorhergehenden Ansprüche, bei dem das Gußpolymer ein SCC-Polymer umfasst.

8. Gußstück nach einem der vorhergehenden Ansprüche 1 bis 6, bei dem das Gußpolymer Polycaprolacton umfasst.

9. Verfahren zur Herstellung eines orthopädischen Gußstücks gemäß einem der Ansprüche 1 bis 8, bei dem die Gußzusammensetzung, während sie geschmolzen ist, auf den Textilträger aufgebracht wird.

10. Verfahren nach Anspruch 9, bei dem zwei oder mehr poröse Lagenmaterialien miteinander laminiert werden.

## Revendications

1. Moulage thermoreformable qui comprend :
(1) un support de tissu reformable qui est dans un état étiré ; et
(2) une composition de moulage rigide qui :
(a) maintient le support dans son état étiré, et
(b) comprend un polymère de moulage ayant un point de fusion cristalline Tₘ ;
ce par quoi le moulage
(A) à des températures inférieures à Tₘ a une première configuration stable,
(B) lorsqu'il est chauffé à une température supérieure à Tₘ en l'absence de contrainte, devient moulable et se reforme de telle sorte qu'au moins une dimension de celui-ci diminue d'une première valeur x à une seconde valeur y qui est d'au plus 0,95.x ; et
(C) lorsqu'il est refroidi à une température inférieure à Tₘ après avoir été moulé, adopte une seconde configuration stable.

2. Moulage selon la revendication 1, dans lequel le support de tissu est un tissu tricoté qui comprend un fil élastomère et un fil non élastomère.

3. Moulage selon la revendication 2, dans lequel le tissu a une puissance, mesurée comme défini dans ce qui précède, de 18 à 360 g/cm (0,1 à 2,0 livres/pouce).

4. Moulage selon l'une quelconque des revendications précédentes, au moins une partie du moulage ayant une pluralité d'ouvertures à travers son épaisseur, chacune des ouvertures ayant une surface de 0,01 à 0,08 cm².

5. Moulage selon l'une quelconque des revendications précédentes, au moins une partie du moulage ayant une perméabilité à l'écoulement d'air, mesurée comme défini dans ce qui précède, de 10 à 35%.

6. Moulage selon l'une quelconque des revendications précédentes, dans lequel la Tm du polymère de moulage est de 45 à 60°C.

7. Moulage selon l'une quelconque des revendications précédentes, dans lequel le polymère de moulage comprend un polymère SCC.

8. Moulage selon l'une quelconque des revendications 1 à 6, dans lequel le polymère de moulage comprend la polycaprolactone.

9. procédé de fabrication d'un moulage orthopédique tel que défini à l'une quelconque des revendications 1 à 8, qui comprend l'application de la composition de moulage, alors qu'elle est fondue, sur le support de tissu.

10. Procédé selon la revendication 9, qui comprend l'opération consistant à laminer ensemble deux matières en feuille poreuses ou davantage.
